# EUROPEAN PATENT APPLICATION

(11) **EP 4 520 255 A1**
(43) Date of publication of application: **12.03.2025**
(21) Application number: 23898209.4
(22) Date of filing: 24.11.2023
(51) Int. Cl.: A61B 5/00, A61B 5/16, G16H 10/20, G16H 10/60, G16H 50/20

(54) **APPARATUS, METHOD, AND COMPUTER PROGRAM FOR ANALYSIS OF COGNITIVE ABILITY WITH RESPECT TO SOCIAL SITUATION BASED ON FEEDBACK ON CONTENT**

(30) Priority: 29.11.2022 KR 20220163318
(71) Applicant: Neudive Inc., Daegu 41061 (KR)
(72) Inventor: JU, Ran, Incheon 21056 (KR); CHO, Sung Ja, Seoul 08727 (KR)
(74) Representative: Krauns, Christian
(86) International application number: PCT/KR2023/019054
(87) International publication number: WO 2024/117678

(57) **Abstract**

A cognitive ability analysis device according to an embodiment may include an information acquisition unit that acquires user information; a content unit that determines the type of content that determines cognitive ability for social situations based on the user information; a query unit that presents the content and a question related to the content to a user and acquires a user's answer; and a determination unit that determines user's cognitive ability for social situations based on the user's answer.

## Description

### TECHNICAL FIELD

The present invention relates to a device, method, and computer program for analyzing cognitive ability for social situations based on content feedback.

### BACKGROUND ART

Autism refers to a disorder in which a person does not form relationships with others and does not develop emotional bonds. The International Classification of Diseases (ICD-10) diagnosed autism as a pervasive developmental disorder throughout development, and categorized pervasive developmental disorders into nine subtypes. Recently, a follow-up study related to this concluded that it is not clinically significant to categorize them separately, and autism is also referred to as the autism spectrum.

Meanwhile, in determining whether a child has autism, a model has been used to check whether the child shows an appropriate level of emotional response to a given specific situation, and to diagnose autism if an appropriate level of response is not shown.

However, because the factors that need to be determined in order to recognize various social situations may vary not only in emotional responses but also in the patterns of social situations, an accurate assessment tool is needed to implement a social skills training program that allows children with autism to accurately recognize and cope with social situations.

### DESCRIPTION OF EMBODIMENTS

### TECHNICAL PROBLEM

Provided is a technology capable of analyzing various user feedback on provided content in relation to a social situation context to determine an accurate cognitive state for social situations.

However, the problems to be solved by the present invention are not limited to those mentioned above, and may include purposes that are not mentioned but can be clearly understood by one of ordinary skill in the art to which the present invention belongs from the following description.

### SOLUTION TO PROBLEM

A cognitive ability analysis device according to an embodiment may include an information acquisition unit that acquires user information; a content unit that determines the type of content that determines cognitive ability for social situations based on the user information; a query unit that presents the content and a question related to the content to a user and acquires a user's answer; and a determination unit that determines user's cognitive ability for social situations based on the user's answer.

In addition, the user information may include gender, disability level, IQ, age, and Social Responsiveness Scale Second Edition (SRS-2) score.

In addition, the content unit may determine a content level based on the gender, disability level, IQ, age, and SRS-2 score included in the user information.

In addition, the query unit may acquire a first response, which is a response option selected by a user for a question given in the content, a second response to a nonverbal expression that should be performed for a situation given in the content, and a third response to a time when the nonverbal expression should be performed, from the user.

In addition, the determination unit may search for content to be presented to the user based on scores for the first to third responses and provide the content to the user.

In addition, the content may be pre-designated as an area that serves as a clue for determining social situations based on coordinate information for an area that includes some of texts to be output, or coordinate information for an area that includes some of images output by the content.

In addition, the device may further include a gaze acquisition unit that acquires coordinate information of a point where a user gazes at the content, time information of the point of gaze, and information about an order in which the point of gaze has moved in time series.

In addition, the gaze acquisition unit may separately specify and store a point where the user gazes at a certain point for a preset time or longer.

In addition, the determination unit may determine whether the point at which the gaze duration is equal to or greater than the preset time is included within the area serving as a clue.

In addition, the determination unit may determine cognitive ability for social situations of the content from user's gaze information based on a preset score criterion for each of gaze coordinate information, gaze time, whether gaze occurred for a preset time or longer, and whether gaze occurred in an area that serves as a clue.

A cognitive ability analysis method performed by a cognitive ability analysis device according to an embodiment may include acquiring user information; determining the type of content that determines cognitive ability for social situations based on the user information; presenting the content and a question related to the content to a user and acquiring a user's answer; and determining user's cognitive ability for social situations based on the user's answer.

### ADVANTAGEOUS EFFECTS OF DISCLOSURE

According to an embodiment of the present invention, in order to determine an accurate cognitive state for social situations, a sophisticated technique may be provided that analyzes various user feedback on provided content in relation to a social situation context. For example, in cases where group bullying occurs or when several people create a deceptive situation to tease one person, it is impossible to accurately recognize the overall situation by looking at only one person's facial expression, so the present invention may determine user's cognitive ability for social situations based on whether a user shows any reaction in a situation created by content. As a result, the present invention may serve as an evaluation tool for implementing a social skills training program that enables a user to accurately recognize and cope with various social situations.

Effects obtainable in the present invention are not limited to the above-mentioned effects, and other effects not mentioned will be clearly understood by one of ordinary skill in the art to which the disclosure belongs from the following description.

### BRIEF DESCRIPTION OF DRAWINGS

FIG. 1 is a functional block diagram of a cognitive ability analysis device for social situations based on feedback of content according to an embodiment of the present invention.
FIG. 2 is an exemplary view of types of content according to an embodiment of the present invention.
FIG. 3 is an exemplary view of a question related to content according to an embodiment of the present invention.
FIG. 4 is an exemplary view of a clue area of content according to an embodiment of the present invention.
FIG. 5 is an exemplary view of gaze information collected from content according to an embodiment of the present invention.
FIG. 6 is an exemplary view of an operation for determining user's cognitive ability for social situations based on user's gaze information and answer according to an embodiment of the present invention.
FIG. 7 is a flowchart of a cognitive ability analysis method performed by the cognitive ability analysis device 100 according to an embodiment of the present invention.

### MODE OF DISCLOSURE

Hereinafter, embodiments of the present invention will be described with reference to the drawings. In the following description, descriptions of a well-known technical configuration in relation to a lead implantation system for a deep brain stimulator will be omitted. For example, descriptions of the configuration/structure/method of a device or system commonly used in deep brain stimulation, such as the structure of an implantable pulse generator, a connection structure/method of the implantable pulse generator and a lead, and a process for transmitting and receiving electrical signals measured through the lead with an external device, will be omitted. Even if these descriptions are omitted, one of ordinary skill in the art will be able to easily understand the characteristic configuration of the present invention through the following description.

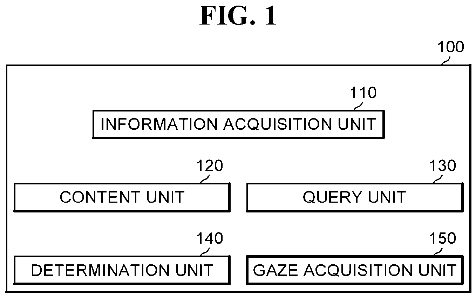

FIG. 1 is a functional block diagram of a cognitive ability analysis device 100 for social situations based on feedback of content (Hereinafter, 'cognitive ability analysis device 100') according to an embodiment of the present invention.

Referring to FIG. 1, the cognitive ability analysis device 100 according to an embodiment of the present invention may include an information acquisition unit 110, a content unit 120, a query unit 130, and a determination unit 140, and may further include a gaze acquisition unit 150. The cognitive ability analysis device 100 according to an embodiment of the present invention may perform overall operations by one or more processors, and the one or more processors may control functional blocks included in FIG. 1 to perform operations described below.

The information acquisition unit 110 may acquire information about a user whose cognitive ability for social situations is tested through content provided by the present invention. The information acquisition unit 110 may directly receive user information from a user, or may acquire user information from a user terminal connected by wire or wirelessly. For example, user information may include user's gender, disability level, IQ, age, and Social Responsiveness Scale Second Edition (SRS-2) score.

The content unit 120 may provide a user with content for assessing cognitive ability for social situations. The content unit 120 may determine the level of content to be provided to a user based on the user information acquired by the information acquisition unit 110. According to an embodiment, content may be classified into levels according to the difficulty of determining social situations, and each content may include an image, text, and voice representing social situations. The content unit 120 may simultaneously output a voice matching text shown in content. For example, the content unit 120 may output content to a user through a display connected to the cognitive ability analysis device 100 by wire or wirelessly. For example, the content unit 120 may provide content to a user terminal connected to the cognitive ability analysis device 100 by wire or wirelessly. The content unit 120 may include a hardware memory for storing content. The content unit 120 may include a communication module for acquiring content from an external device.

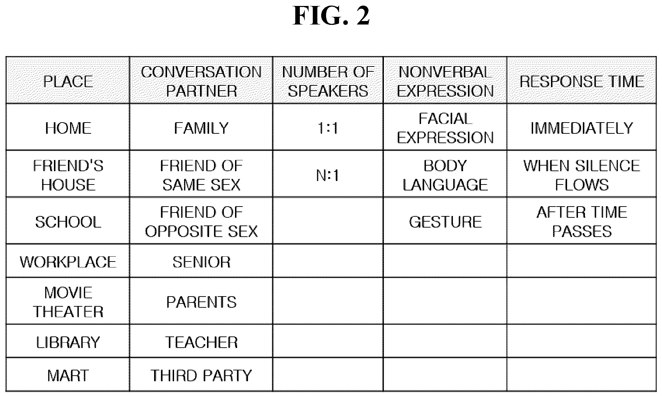

FIG. 2 is an exemplary view of types of content according to an embodiment of the present invention.

Referring to FIG. 2, content according to an embodiment may be classified and stored according to the type of content according to the conversation location, conversation partner, number of speakers, the type of nonverbal expression to be asked to a user, and response time to the nonverbal expression to be asked to the user. The content unit 120 may determine the type of content to be provided to a user based on user information.

The query unit 130 may provide content to a user and acquire a user's answer by presenting a question related to the provided content to the user.

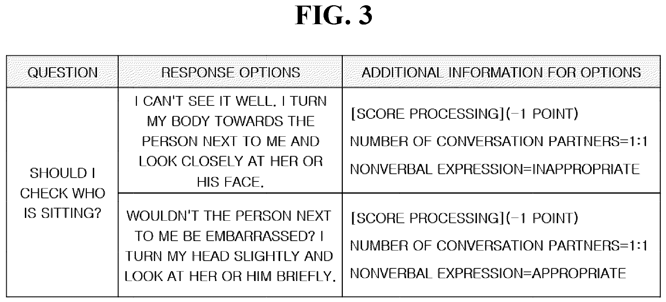

FIG. 3 is an exemplary view of a question related to content according to an embodiment of the present invention.

Referring to FIG. 3, content according to an embodiment may be stored by mapping a related question and response options, and the query unit 130 may calculate a score for user's cognitive ability based on user's selection according to the response options.

For example, the query unit 130 may acquire a first response, which is a response option selected by a user for a question given in the content, a second response to a nonverbal expression that should be performed for a situation given in the content, and a third response to a time when a nonverbal expression should be performed, from the user.

The determination unit 140 may determine user's cognitive ability for social situations based on a user's answer. The determination unit 140 may determine the level of next content to be presented to a user based on scores mapped to the first response, the second response, and the third response acquired by the query unit 130.

According to an embodiment further including the gaze acquisition unit 150, the gaze acquisition unit 150 may acquire gaze information of a point where a user gazes at content.

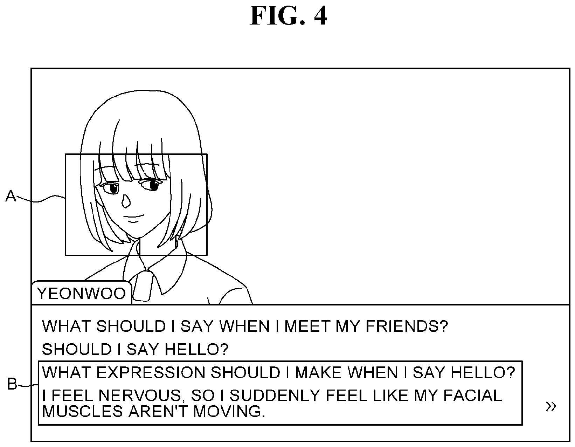

FIG. 4 is an exemplary view of a clue area of content according to an embodiment of the present invention.

Referring to FIG. 4, according to an embodiment, content may have areas (A and B in FIG. 4) pre-designated as clues for determining social situations. For example, in order to specify the areas, content may have coordinate information pre-specified within an area (A in FIG. 4) that includes some images (e.g., areas of images expressing facial expressions) from among areas where images of the content are output, or coordinate information pre-specified within an area (B in FIG. 4) that includes some texts (e.g., areas of text expressing emotions) from among areas where text of the content is output.

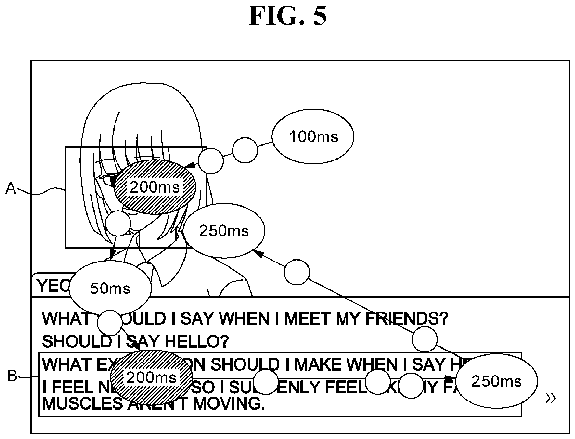

FIG. 5 is an exemplary view of gaze information collected from content according to an embodiment of the present invention.

Referring to FIG. 5, the gaze acquisition unit 150 may acquire coordinate information of a point where a user gazes at the content, time information of the point of gaze, and information about an order in which the point of gaze has moved in time series. For example, the gaze acquisition unit 150 may include a camera module capable of detecting a user's gaze, and may measure coordinate information of a point where a user gazes in units of 10 ms and a gaze retention time of the user at a certain point. The gaze acquisition unit 150 may separately specify and store a point where a user gazes at a certain point for a preset time (e.g., 60 ms) or longer, and at this time, the determination unit 140 may determine whether areas that serve as clues (e.g., A and B in FIG. 4) include the point where a user gazes at a certain point for a preset time (e.g., 60 ms) or longer. The gaze acquisition unit 150 may acquire information about an order in which a user's gaze has moved in time series by storing acquired time information in time series.

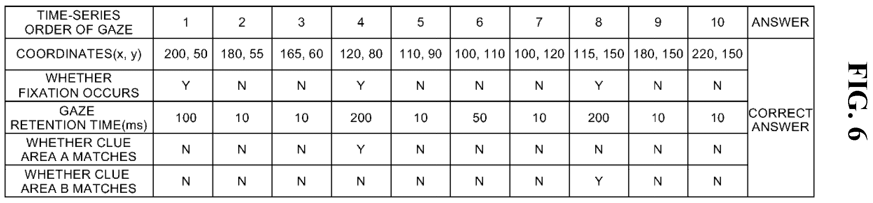

FIG. 6 is an exemplary view of an operation for determining user's cognitive ability for social situations based on user's gaze information and answer according to an embodiment of the present invention.

Referring to FIG. 6, the determination unit 140 may determine cognitive ability for social situations of content from the user's gaze information based on a preset score criterion for each of gaze coordinate information, whether gaze occurred for a preset time (e.g., 60 ms) or longer, gaze time, and whether gaze occurred in areas that serve as clues (e.g., A and B in FIG. 4) according to a time-series order of the user's gaze. In addition, the determination unit 140 may determine the user's cognitive ability for social situations from the user's gaze information by using a neural network model trained to determine cognitive ability for social situations based on each data such as gaze coordinate information, gaze time, whether gaze occurred for a preset time or longer, and whether gaze occurred in an area that serves as a clue.

For example, when a user's answer to a question related to content is correct, but a user's cognitive ability score determined from user's gaze information is below a preset value, the determination unit 140 may determine that the user's answer is incorrect and search for new content to reconfirm user's cognitive ability to re-measure the user's cognitive ability.

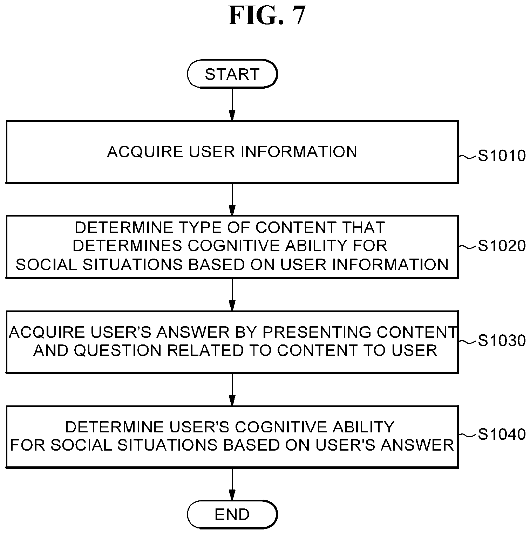

FIG. 7 is a flowchart of a cognitive ability analysis method performed by the cognitive ability analysis device 100 according to an embodiment of the present invention. Each operation of the cognitive ability analysis method according to FIG. 7 may be performed by the cognitive ability analysis device 100 described in FIG. 1, and each operation is described as follows.

In operation S1010, the information acquisition unit 110 may acquire user information.

In operation S1020, the content unit 120 may determine the type of content that determines cognitive ability for social situations based on the user information.

In operation S1030, the query unit 130 may acquire a user's answer by presenting content and a question related to the content to a user.

In operation S1040, the determination unit 140 may determine user's cognitive ability for social situations based on the user's answer.

Meanwhile, in addition to the operations shown in FIG. 7, because the information acquisition unit 110, the content unit 120, the query unit 130, the determination unit 140, and the gaze acquisition unit 150 described above configure various embodiments to perform the operations described in FIGS. 1 to 6, even in the operations of FIG. 7, a new operation performed by each functional block may be added. Because a configuration of an additional operation and operations of components responsible for each operation to perform respective operations have been described in FIGS. 1 to 6, redundant descriptions will be omitted.

According to the embodiment described above, in order to determine an accurate cognitive state for social situations, a sophisticated technique may be provided that analyzes various user feedback on provided content in relation to a social situation context. For example, in cases where group bullying occurs or when several people create a deceptive situation to tease one person, it is impossible to accurately recognize the overall situation by looking at only one person's facial expression, so the present invention may determine user's cognitive ability for social situations based on whether a user shows any reaction in a situation created by content. As a result, the present invention may serve as an evaluation tool for implementing a social skills training program that enables a user to accurately recognize and cope with various social situations.

In concluding the detailed description, those skilled in the art will appreciate that many variations and modifications may be made to the preferred embodiments without substantially departing from the principles of the present invention. Therefore, the disclosed preferred embodiments of the invention are used in a generic and descriptive sense only and not for purposes of limitation.

## Claims

1. A cognitive ability analysis device comprising:
an information acquisition unit that acquires user information;
a content unit that determines a type of content that determines cognitive ability for social situations based on the user information;
a query unit that presents the content and a question related to the content to a user and acquires a user's answer; and
a determination unit that determines user's cognitive ability for social situations based on the user's answer.

2. The cognitive ability analysis device of claim 1, wherein the user information comprises gender, disability level, IQ, age, and Social Responsiveness Scale Second Edition (SRS-2) score.

3. The cognitive ability analysis device of claim 2, wherein the content unit determines a content level based on the gender, disability level, IQ, age, and SRS-2 score included in the user information.

4. The cognitive ability analysis device of claim 1, wherein the query unit acquires a first response, which is a response option selected by a user for a question given in the content, a second response to a nonverbal expression that should be performed for a situation given in the content, and a third response to a time when the nonverbal expression should be performed, from the user.

5. The cognitive ability analysis device of claim 4, wherein the determination unit searches for next content to be presented to the user based on scores for the first to third responses and provide the content to the user.

6. The cognitive ability analysis device of claim 1, wherein the content is pre-designated as an area that serves as a clue for determining social situations based on coordinate information for an area that includes some of texts to be output, or coordinate information for an area that includes some of images output by the content.

7. The cognitive ability analysis device of claim 6, further comprising:
a gaze acquisition unit that acquires coordinate information of a point where a user gazes at the content, time information of the point of gaze, and information about an order in which the point of gaze has moved in time series.

8. The cognitive ability analysis device of claim 7, wherein the gaze acquisition unit separately specifies and stores a point where the user gazes at a certain point for a preset time or longer.

9. The cognitive ability analysis device of claim 8, wherein the determination unit determines whether the point at which the gaze duration is equal to or greater than the preset time is included within the area serving as a clue.

10. The cognitive ability analysis device of claim 9, wherein the determination unit determines cognitive ability for social situations of the content from user's gaze information based on a preset score criterion for each of gaze coordinate information, gaze time, whether gaze occurred for a preset time or longer, and whether gaze occurred in an area that serves as a clue.

11. A cognitive ability analysis method performed by a cognitive ability analysis device, the cognitive ability analysis method comprising:
acquiring user information;
determining a type of content that determines cognitive ability for social situations based on the user information;
presenting the content and a question related to the content to a user and acquiring a user's answer; and
determining user's cognitive ability for social situations based on the user's answer.

12. A program stored on a computer-readable recording medium for executing each operation according to the method of claim 11.
